# EUROPEAN PATENT APPLICATION

(11) **EP 4 197 547 A1**
(43) Date of publication of application: **21.06.2023**
(21) Application number: 21216132.7
(22) Date of filing: 20.12.2021
(51) Int. Cl.: A61K 35/15, A61P 9/14, A61K 35/17, C12N 5/078, C12N 5/0786

(54) **COMPOSITION FOR TREATING OR PREVENTING VASCULITIS AND DISEASES ASSOCIATED WITH VASCULITIS**

(71) Applicant: Aposcience AG, 1090 Wien (AT)
(72) Inventor: Ankersmit, Hendrik Jan, 1060 Wien (AT); Mildner, Michael, 3040 Neulengbach (AT)
(74) Representative: Schwarz & Partner Patentanwälte GmbH

(57) **Abstract**

The present invention relates to a composition comprising a supernatant of a peripheral blood mononuclear cell (PBMC) cell culture for use in the treatment or prevention of vasculitis and diseases associated with vasculitis.

## Description

### TECHNICAL FIELD

The present invention relates to compositions and methods for treating and preventing vasculitis and diseases associated with vasculitis.

### BACKGROUND ART

Vasculitis is a group of rare diseases of blood vessels including arteries, veins and lymphatic vessels, leading to their destruction. Neutrophils represent one of the key drivers of vasculitis, affecting endothelial cells by release of neutrophil extracellular traps (NETs). There are many types of vasculitis, and they may vary greatly in symptoms, severity and duration. Most types of vasculitis are rare and the causes are generally not known. Vasculitis affects people of both sexes and all ages.

Vasculitis is a systemic disease affecting virtually every organ system, including skin, lungs, joints, kidneys, gastrointestinal tract, blood, eyes, brain, nerves, sinuses, nose and throat. The pattern of organ involvement is specific to the individual as well as the type of vasculitis.

Treatment of vasculitis focuses mainly on controlling the inflammation and managing any underlying conditions that may be triggering the vasculitis. Current therapies are typically based on corticosteroids, such as prednisone. Side effects of corticosteroids can be severe, especially if taken over a long period of time. Possible side effects include weight gain, diabetes and weakened bones. Other medications may be prescribed with corticosteroids to control the inflammation so that the dosage of corticosteroids can be tapered more quickly. The medication used depends on the type of vasculitis that is present. These medications may include methotrexate, azathioprine, mycophenolate, cyclophosphamide, tocilizumab or rituximab. Sometimes, vasculitis causes an aneurysm. Aneurysm may need surgery to reduce the risk of it rupturing. Blocked arteries also may require surgical treatment to restore blood flow to the affected area.

All these medical interventions have side effects and/or bear a certain risk to the patients' health. Furthermore, they are all known to target tissue inflammation, mainly by affecting T-cells function, thereby inhibiting neutrophil recruitment to the endothelium. Treatment options either preventing the release of NETs, or protecting the endothelium are so far not available. Hence, it is an object of the present invention to provide alternative means and methods for treating or preventing vasculitis and diseases associated thereto.

### SUMMARY OF THE INVENTION

It turned surprisingly out that a composition comprising a supernatant of a PBMC cell culture can be used in the treatment or prevention of vasculitis and diseases associated with vasculitis.

In recent years, cell-based therapies have made tangible progress in modern regenerative medicine to either repair injured tissues and organs, restore their functions or replace damaged cells. With the ability for self-renewal, the potential to differentiate into several distinct cell types and numerous successful preclinical studies, regenerative therapy using mesenchymal stem cells (MSCs) has been considered as a highly promising approach. In spite of encouraging pre-clinical data, most of the first-in-men clinical trials failed to show effectivity in patients. With regard to the mode of action (MOA) of MSCs, it has become more and more evident that paracrine factors released from the injected cells are involved in many of the beneficial biological effects and thus contribute significantly to tissue regeneration. As stem cell secretome-based therapies have considerable limitations, i.e. low abundance of cells, invasive procedures to obtain the cells and high costs, alternative sources for cell secretomes with regenerative capacity are highly demanded.

In the last years, several studies suggested the secretome of irradiated peripheral blood mononuclear cells (APOSEC) as a valuable alternative to MSCs. APOSEC displays comparable tissue regenerative properties as compared to paracrine factors released from stem cells, and is more easily obtainable (Ankersmit, H.J. et al. Eur. J. Clin. Invest. 39(2009):445-456). In-depth functional analyses of APOSEC revealed proteins, lipids and extracellular vesicles as the three main biological constituents. Their MOAs are very diverse and have been examined in many pre-clinical experimental setups of induced tissue damage.

Cells, in particular mammalian cells, are known to secrete numerous substances during cultivation into a cell culture medium. The so obtained conditioned culture media can be used in the treatment and/or prevention of various diseases and disorders. For instance, WO 2010/070105 and WO 2010/079086 disclose conditioned culture media ("supernatants") which are obtained by cultivation of PBMCs and which can be used in the treatment of various inflammatory conditions. Hence, "a supernatant of a peripheral blood mononuclear cell (PBMC) cell culture", as used herein, refers to any supernatant obtainable by cultivating PBMCs *in vitro* in a culture medium. After the cultivation step the cultivated PBMCs are removed from the culture medium in order to obtain the substantially cell-free, preferably completely cell-free, supernatant. The supernatant of the PBMC culture comprises next to components of the cultivation medium substances produced and secreted by the PBMCs and/or even lysed PBMCs. "Supernatant" can be used interchangeable with conditioned culture medium obtainable by cultivating PBMCs.

It is a surprising finding that the supernatants of the present invention are able to inhibit or even prevent the release of NETs which are involved in the formation of vasculitis. It is also surprising that the supernatant of the present invention shows endothelium protecting effects due to its protease inhibitory activity. This supports the maintenance of blood vessel integrity and function to prevent and/or treat vasculitis.

### BRIEF DESCRIPTION OF THE FIGURES

Fig. 1 shows that APOSEC inhibits serine proteases and blocks thrombin-induced decrease of the endothelial barrier function *in vitro.*
Fig. 2 shows that Aposec prevents NET-induced endothelial tissue damage.

### DESCRIPTION OF EMBODIMENTS

The terms "preventing" and "prevention", as used herein, refer to the prevention or inhibition of the recurrence, onset and development of vasculitis and/or diseases associated with vasculitis in a human and mammalian body resulting from the administration of the supernatant according to the present invention. In some embodiments "preventing" and "prevention" refers to the reduction of the risk to develop vasculitis and/or diseases associated with vasculitis. The term "preventing" covers measures not only to prevent the occurrence of vasculitis and/or diseases associated with vasculitis, but also to arrest its progress and reduce its consequences once established.

The terms "treatment" and "treating", as used herein, refer to the reduction or inhibition of the progression and duration of vasculitis and/or diseases associated with vasculitis, the reduction or amelioration of the severity of the vasculitis and/or diseases associated with vasculitis and the amelioration of one or more symptoms thereof. "Treatment" encompasses also the improvement and/or reversal of the symptoms of vasculitis and/or diseases associated with vasculitis. The term "treatment" refers to both therapeutic treatment and prophylactic measures. For example, those who may benefit from treatment with compositions and methods of the present invention include those already with vasculitis and/or diseases associated with vasculitis as well as those in which the vasculitis and/or diseases associated with vasculitis are to be prevented.

According to a preferred embodiment of the present invention the diseases associated with vasculitis of small, medium and large blood vessels are selected from the group consisting of vasculitis in the ear-nose-throat (ENT), preferably granulomatous and/or necrotizing lesions of the ENT-tract, pulmonary haemorrhage, haemoptysis, dermal vasculitides, glomerulonephritis, proteinuria, haematuria, focal vasculitis, eosinophilic myocarditis, cardiomyopathy and virus-associated vasculitis.

According to a further preferred embodiment of the present invention the PBMCs are cultivated in a cell culture medium selected from the group consisting of a cell growth medium, preferably CellGro medium, more preferably Cellgro GMP DC medium, RPMI, DMEM, X-vivo and Ultraculture.

According to another preferred embodiment of the present invention the PBMCs of the PBMC cell culture are subjected to one or more stress inducing conditions before or during cultivation.

The term "under stress inducing conditions", as used herein, refers to cultivation conditions leading to stressed cells. Conditions causing stress to cells include among others radiation, in particular ionizing radiation or UV radiation, heat, chemicals, radiation, hypoxia, osmotic pressure, pH shift etc.

According to a preferred embodiment of the present invention the stress inducing conditions include hypoxia, ozone, heat (e.g. more than 2°C, preferably more than 5°C, more preferably more than 10°C, higher than the optimal cultivation temperature of PBMCs, i.e. 37°C), radiation (e.g. UV radiation, gamma radiation), chemicals, osmotic pressure (i.e. osmotic conditions which are decreased at least by 10% in comparison to osmotic conditions regularly occurring in a body fluid, in particular in blood) or combinations thereof.

Hence, according to a further preferred embodiment of the present invention the stress inducing condition is selected from the group consisting of radiation, in particular ionizing radiation or UV radiation, hypoxia, ozone, heat, osmotic pressure and pH shift.

According to another preferred embodiment of the present invention the PBMCs are subjected to an ionizing radiation, preferably gamma radiation, at a dose of at least 10 Gy, preferably at least 20 Gy, more preferably at least 30 Gy, more preferably at least 40 Gy, more preferably at least 50 Gy.

According to a preferred embodiment of the present invention the PBMCs are cultivated for at least 4 h, preferably for at least 6 h, more preferably for at least 12 h, before isolating its supernatant.

According to a preferred embodiment of the present invention the PCBMC cell culture comprises 1×10⁵ to 1×10⁸ PBMCs/ml, preferably 1×10⁶ to 1×10⁷ PBMCs/ml, more preferably 2×10⁶ to 25×10⁶ PBMCs/ml.

It turned out that the composition of the supernatant obtainable by cultivating PBMCs shows advantageous properties if a certain amount of PBMCs per ml cell culture medium is cultivated.

According to another preferred embodiment of the present invention 0.1 to 5 ml supernatant/kg body weight, preferably 0.3 to 3 ml/kg body weight, more preferably 0.5 to 2 ml/kg body weight, more preferably 0.8 to 1.2 ml/kg body weight, is administered to a human or animal mammalian body.

The composition of the present invention comprises a supernatant in an amount sufficient to treat or prevent vasculitis and diseases associated with vasculitis. The volume of supernatant administered per kg body weight to a human or mammal body as indicated above refers directly to the supernatant. If the volume is too large to be administered to a human or mammal body, this volume can be reduced by, e.g., lyophilisation. Hence, the volume of the composition of the present invention to be administered may be lower than that indicated for the supernatant. A person skilled in the art knows which volumes can be administered using a specific administration route.

According to a preferred embodiment of the present invention the composition is administered by inhalation, topically, orally, sublingually, buccally, subcutaneously or intravenously, whereby it is most preferred to administer the composition of the present invention intravenously.

The composition of the present invention may comprise pharmaceutically acceptable excipients such as diluents, stabilizers, carriers etc. Depending on the dosage form the preparation according to the present invention comprises the respective ingredients. Methods for preparing the same are well known to the skilled artisan.

In order to increase the shelf-life of the composition according to the present invention the supernatant or even the complete composition may be lyophilised. Methods for lyophilising such preparations are well known to the person skilled in the art.

Prior its use the lyophilised preparation can be contacted with water or an aqueous solution comprising buffers, stabilizers, salts etc.

According to another preferred embodiment of the present invention the mammal is a horse, a dog, a cat, or a camel.

The composition of the present invention can be used to treat any kind of mammal. However, the aforementioned mammals are most preferred.

Another aspect of the present invention relates to a method for treating or preventing vasculitis and diseases associated with vasculitis comprising the step of administering a composition as defined above.

### EXAMPLE

The present example shows that APOSEC protects blood vessels inflammation and damage by a dual mechanism: 1) APOSEC inhibits the induction of NETs, thereby preventing NETs-induced vasculitis, and 2) APOSEC directly prevents vascular leakage.

### Materials and Methods

### PBMC isolation and generation of PBMC secretome

APOSEC was produced in compliance with good manufacturing practice (GMP) by the Austrian Red Cross, Blood Transfusion Service for Upper Austria (Austria). Briefly, PBMCs were obtained by Ficoll-Paque PLUS (GE Healthcare, USA)-assisted density gradient centrifugation and exposed to 60 Gy Caesium 137 gamma-irradiation (IBL 437C, Isotopen Diagnostik CIS GmbH, Germany). Cells were adjusted to a concentration of 2.5×10⁷ cells/mL (equates to 25 U/ml) and cultured in phenol red-free CellGenix GMP DC medium (CellGenix GmbH, Germany) for 24 hours. Cells and cellular debris were removed by centrifugation and supernatants were passed through a 0.2 µm filter. For viral clearance, methylene blue treatment was performed. The secretome was lyophilized, terminally sterilized by high-dose gamma-irradiation, and cryopreserved.

### Protease activity assays

To test the inhibitory effects of APOSEC on protease activity a fluorometric enzyme activity assay (Enzcheck) using the unselective serine protease trypsin (Gibco) at a concentration of 0.05% was performed. PBMCsec stock concentration was set at 12.5 U/ml, according to the manufacturers' instructions.

### Transendothelial electrical resistance (TEER)

Changes in electrical impedance of dermal microvascular endothelial cells (DMEC) were measured using the ECIS system (Applied Biophysics). A total of 4000 cells/cm² were seeded in each well of an ECIS chamber (ibidi GmbH, Germany). Once they reached full confluency cells were treated with Thrombin and Thrombin in combination with control medium (concentrated at 12,5 U/ml) or APOSEC (concentrated at 12,5 U/ml), respectively. Endothelial resistance was monitored at 250 Hz over 2 hours following the addition of treatments, according to a previously published protocol (Schossleitner, K. et al. Arter. Thromb Vasc Biol 36(2016):647-654).

### Co-culture of endothelial cells and neutrophils

Neutrophils were isolated from healthy donors and stimulated with Ionomycin to induce NET-formation in presence or absence of APOSEC. The cells were incubated with their respective treatment for one hour at 37°C followed by washing with PBS prior to the addition to primary human umbilical vein endothelial cells (HUVECs). The co-culture was either performed by directly adding neutrophils to a confluent HUVEC monolayer or separating the two cell populations by a transwell-insert providing a physical barrier by a 0,4 µm mesh/filter. After 2 hours, neutrophils were removed and HUVECs were cultured for 24 hours under standard conditions. Cell culture supernatant of HUVECs was obtained and stored at -20°C until further processing.

### ELISA

Enzyme linked immunosorbent assay (ELISA) was performed for common pro-inflammatory cytokines and activation markers associated with injured or damaged endothelial tissue (all R&D Systems, USA) according to the manufacturers' instructions. citH3 ELISA (clone 11D3, Cay501620-96, Caymen) was performed as recommended by the manufacturers.

### Flow cytometry

Flow cytometric assessment was routinely performed on FACSCalibur (BD Biosciences, USA) as recommended by the manufacturer. To assess netosis, triple staining with CD66b - Pacific Blue (Biolegend, USA), CD15 - PE-Cy7 (Biolegend) and citH3 - FITC (Abcam, UK) was performed.

### Results

### APOSEC inhibits serine proteases and blocks protease-induced vascular leakage

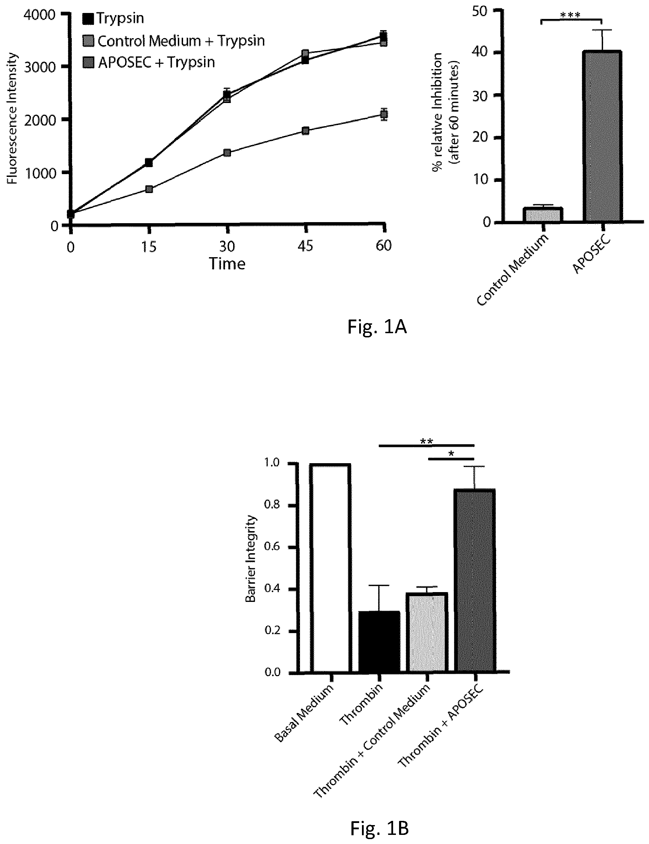

To investigate the potency of APOSEC to inhibit serine protease activity, a protease activity assay on the serine-protease trypsin was performed. While control medium showed a negligible inhibitory effect on protease activity (3.72 %), addition of APOSEC resulted in a 41.96% inhibition of the enzymatic activity of trypsin (Fig. 1A). Next it was investigated whether APOSEC also interferes with thrombin-induced breakdown of the endothelial barrier, as measured by electrical resistance. While basal medium alone showed no effect, addition of thrombin led to a strong drop of the endothelial resistance. This drop was strongly reduced by addition of APOSEC. Addition of control medium showed only a weak effect on the endothelial resistance (Fig. 1B).

In detail, Fig. 1 shows that APOSEC inhibits serine proteases and blocks thrombin-induced decreased of the endothelial barrier function *in vitro.* According to Fig. 1A APOSEC significantly inhibits enzymatic activity of trypsin after 60 minutes when compared to Control Medium (Control Medium 3.72% vs. 41.96% relative inhibition of trypsin activity, p-value = 0.0002). According to Fig. 1B dermal microvascular endothelial cells (DMECs) showed decreased barrier function after treatment with the serine-protease thrombin when compared to Basal Medium (Basal medium 1 vs. 0.29 Thrombin). While the combination of Thrombin with control medium resulted in a comparable decrease (Basal Medium 1 vs. 0.38 Thrombin + Control Medium) addition of APOSEC to thrombin displayed a significantly lower reduction (Basal Medium 1 vs. 87.5 Thrombin + Aposec) and prevents disruption of the endothelial barrier. Barrier function of Thrombin + Aposec is significantly higher when compared to Thrombin (p-value = 0.0071) and Thrombin + Control Medium (p-value = 0.0126)

### APOSEC inhibits NET-formation and NETs-induced activation of endothelial cells

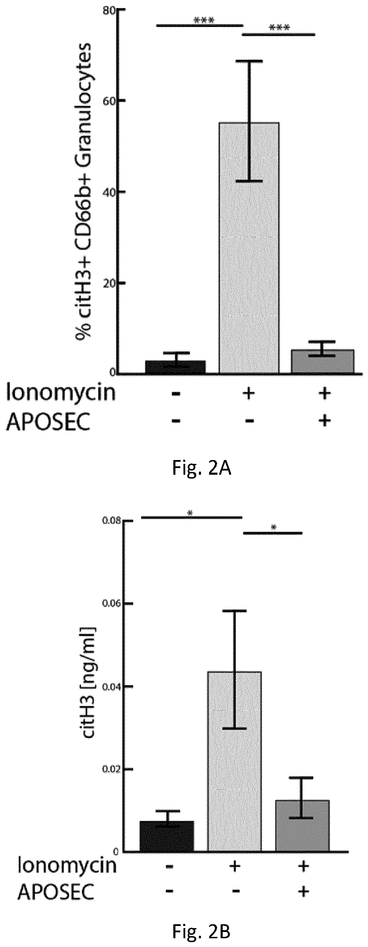

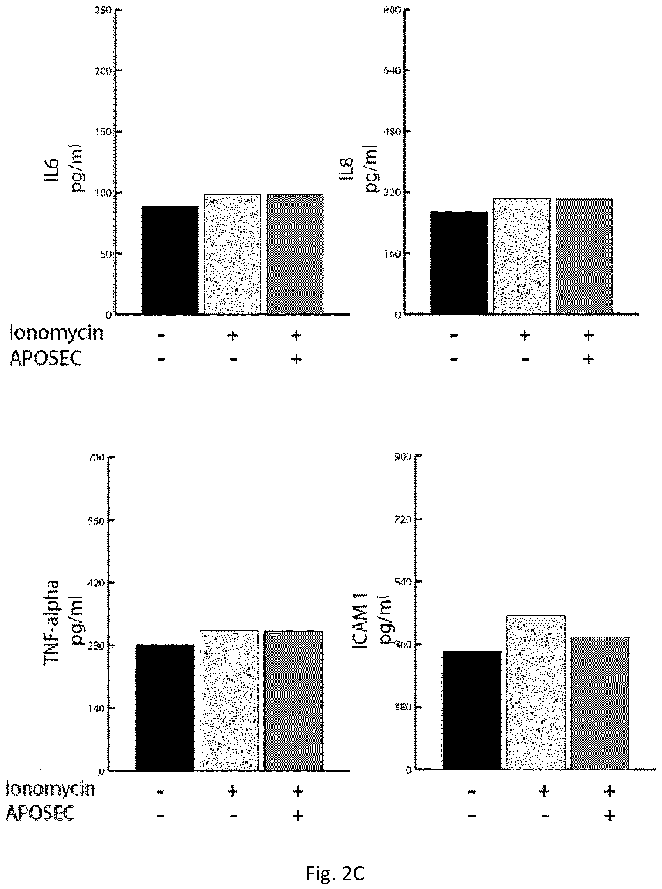

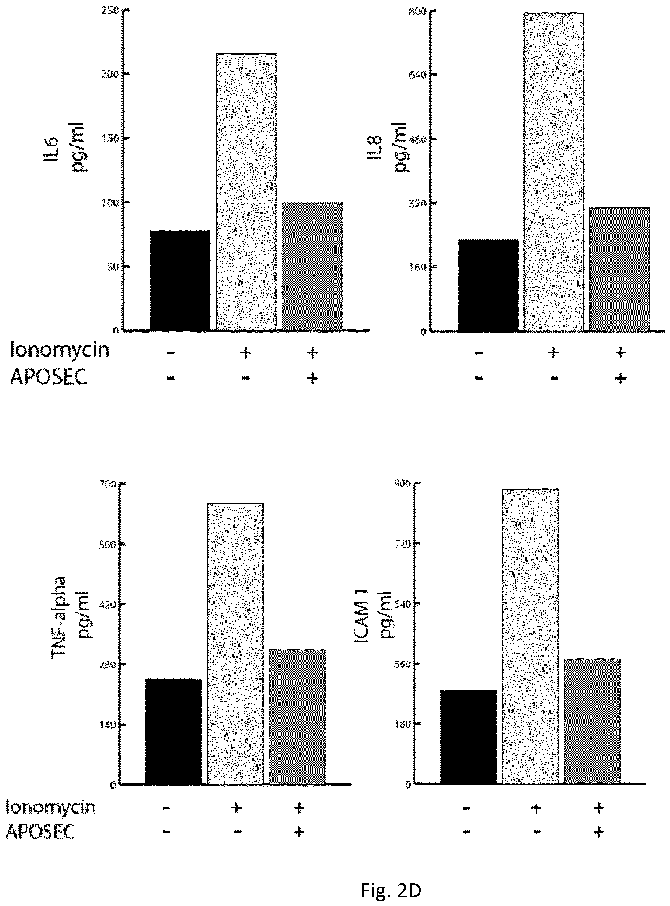

To investigate whether APOSEC has an effect on ionomycin-induced NET-formation, whole blood was stimulated with ionomycin in the absence or presence of APOSEC. Induction of citrul-linated histone-3 (CitH3), a specific marker for netosis, was measured by flow cytometry (Fig. 2A) and ELISA (Fig. 2B). In both assays a strong reduction of ionomycin-induced histone citrullinytion was observed (Fig. 2A and B). Next the effect of APOSEC on NET-induced endothelial tissue damage in an *in vitro* co-culture model was investigated. To assess whether direct contact of activated neutrophils is required for activation and damage of endothelial cells, a co-culture with either direct contact between neutrophils end endothelial cells (Fig 2D), or a physical barrier between them in form of a transwell insert (Fig 2C) was established. The physical barrier appeared to be sufficient to block pro-inflammatory effects as well as tissue damaging capacities of neutrophils on endothelial cells as depicted in Fig. 2C. However, direct contact between ionomycin-activated neutrophils and endothelial cells significantly increased production of pro-inflammatory cytokines, including interleukin (IL)-6, IL-8 and tumor necrosis factor alpha (TNFa), and endothelial activation markers, such as intercellular adhesion molecule 1 (ICAM-1) (Fig. 2D). Treatment of activated neutrophils with APOSEC prior to the addition to endothelial cells almost completely abolished the inflammatory response of endothelial cells, resembling similar cytokine levels as untreated cells (Figure 2D).

Fig. 2 shows that Aposec prevents NET-induced endothelial tissue damage. In Fig. 2A flow cytometry analysis of whole blood samples stimulated with Ionomycin and treated with APOSEC; n=3 are shown. Fig. 2B shows an ELISA of citrulinated histone h3 (citH3) of whole blood samples stimulated with Ionomycin and treated with APOSEC; n=2. Fig. 2C and Fig. 2D show ELISA of pro-inflammatory cytokines and activation markers of HUVECs derived from a neutrophil-HUVEC co-culture with either a transwell-insert as barrier between the cell populations (Fig. 2C) or direct contact (Fig. 2D). Treatment conditions are indicated by - and + below each graph. IL6, Interleukin-6; IL8, Interleukin-8; TNF-alpha, Tumor necrosis factor alpha; ICAM-1, intracellular adhesion molecule 1; - , absent; +, present.

### Conclusion

Together, the above data show that APOSEC is able to reduce blood vessel damage by inhibiting enzymatic destruction of the vessel integrity and by inhibiting NETs-release by neutrophils. These mechanisms are involved in a variety of severe diseases, including any kind of vasculitides, granulomatous or necrotizing lesions of the ENT-tract, pulmonary haemorrhage and haemoptysis, petechial or rash accompanying necrotizing dermal vasculitides, glomerulonephritis and proteinuria, haematuria, focal vasculitis of the vasa nervorum affecting the peripheral nervous system, eosinophilic myocarditis and cardiomyopathy and Covid-19 which might all benefit from the application of APOSEC.

## Claims

1. Composition comprising a supernatant of a peripheral blood mononuclear cell (PBMC) cell culture for use in the treatment or prevention of vasculitis and diseases associated with vasculitis.

2. Composition for the use according to claim 1, wherein the diseases associated with vasculitis of small, medium and large blood vessels are selected from the group consisting of vasculitis in the ear-nose-throat (ENT), preferably granulomatous and/or necrotizing lesions of the ENT-tract, pulmonary haemorrhage, haemoptysis, dermal vasculitides, glomerulonephritis, proteinuria, haematuria, focal vasculitis, eosinophilic myocarditis, cardiomyopathy and virus-associated vasculitis.

3. Composition for the use according to claim 1 or 2, wherein the composition is administered subcutaneously, intramuscularly, subdermally, intradermally or intravenously.

4. Composition for the use according to any one of claims 1 to 3, wherein the PBMC cell culture comprises monocytes, T cells, B cells and/or NK cells.

5. Composition for the use according to any one of claims 1 to 4, wherein the PBMCs are cultivated in a cell culture medium selected from the group consisting of a cell growth medium, preferably CellGro medium, more preferably Cellgro GMP DC medium, RPMI, DMEM, X-vivo and Ultraculture.

6. Composition for the use according to any one of claims 1 to 5, wherein the PBMCs are subjected to one or more stress inducing conditions before or during cultivation.

7. Composition for the use according to claim 6, wherein the stress inducing condition is selected from the group consisting of radiation, in particular ionizing radiation or UV radiation, hypoxia, ozone, heat, osmotic pressure and pH shift.

8. Composition for the use according to claim 7, wherein the PMCs are subjected to an ionizing radiation at a dose of at least 10 Gy, preferably at least 20 Gy, more preferably at least 30 Gy, more preferably at least 40 Gy, more preferably at least 50 Gy.

9. Composition for the use according to any one of claims 1 to 8, wherein the PBMCs are cultivated for at least 4 h, preferably for at least 6 h, more preferably for at least 12 h, more preferably for at least 24 h before isolating its supernatant.

10. Composition for the use according to any one of claims 1 to 9, wherein the PCBMC cell culture comprises 1×10⁵ to 1×10⁸ PBMCs/ml, preferably 1×10⁶ to 1×10⁷ PBMCs/ml, more preferably 2×10⁶ to 25×10⁶ PBMCs/ml.
